# EUROPEAN PATENT SPECIFICATION

(11) **EP 4 298 345 B1**
(45) Date of publication and mention of the grant of the patent: **10.12.2025**
(21) Application number: 21713190.3
(22) Date of filing: 23.02.2021
(51) Int. Cl.: F04B 53/10

(54) **PUMP-VALVING ASSEMBLY FOR A PULSATILE FLUID PUMP**
PUMPEN-VENTIL-ANORDNUNG FÜR EINE PUMPE MIT PULSIERENDER FLÜSSIGKEIT
ENSEMBLE POMPE-SOUPAPE POUR POMPE À FLUIDE PULSATILE

(43) Date of publication of application: 03.01.2024
(73) Proprietor: Ventriflo, Inc., Pelham, NH 03076 (US)
(72) Inventor: VINCENT, Douglas E., Pelham, New Hampshire 03076 (US); KOENIG, George, Pelham, New Hampshire 03076 (US); MURPHY, Matthew J., Marshfield, MA 02050 (US)
(74) Representative: Potter Clarkson
(86) International application number: PCT/US2021/019238
(87) International publication number: WO 2022/182331

(56) References cited:
- WO-A1-98/36792
- FR-A1- 2 309 206
- US-A1- 2007 255 089
- US-A1- 2020 208 503

## Description

### Technical Field

The present invention relates to pulsatile fluid pumps, and more particularly to pulsatile fluid pumps suitable for pumping blood.

### Background Art

A pulsatile fluid pump is taught in U.S. patent 7,850,593 ("our prior patent") for an invention of Douglas Vincent and Matthew Murphy, who are co-inventors of the present invention. Our prior patent discloses a pump actuated by a linear motor configured to cause reciprocation of a flexible membrane, serving as a wall of a fluid housing, that is in turn coupled to a pair of ball valves, in a manner as to implement pulsatile fluid flow.

US 2007/255089 discloses a pumping system providing a physiological pulsatile flow and including controller, a pump drive head coupled to a motor and a fluid housing having at least one port. The port includes a ball valve retainer region, a valve seat, and an occluder ball disposed in the ball valve retainer region 69. During operation, the motor forces the fluid in and out the fluid housing and causes the occluder ball to move from a first position whereby the fluid cannot pass through the port, to a second position whereby the fluid moves annular to and generally around the occluder ball. This movement creates a slight flow reversal that "breaks up" any blood clots that may form. The pumping system may be used as part of a cardiopulmonary bypass system, a ventricular assist device (VAD) and/or a heart pump.

WO 98/36792 discloses a ball valve for a ventricular assist device wherein the ball valve includes a casing. The casing can include ribs to provide a guide surface as the ball occluder moves between a valve closed position and a valve open position. In a particular form, in its open position the centre line of the ball occluder is offset from the valve casing centre line.

### Summary of the Embodiments

In accordance with one embodiment of the invention, which is defined by the appended claims, a pump-valving assembly for a pulsatile fluid pump includes a generally cylindrical pumping chamber, an inlet port having an entrance and coupled to the pumping chamber, and an outlet port having an exit and coupled to the pumping chamber. The pump-valving assembly further includes a spherically shaped inlet ball check-valve assembly coupled to the inlet port, first and second tapered tracts coupled to the inlet ball check-valve assembly and disposed between the inlet port and the pumping chamber respectively, a spherically shaped outlet ball check-valve assembly coupled to the outlet port, and third and fourth tapered tracts coupled to the outlet ball check-valve assembly and disposed between the pumping chamber and outlet port respectively. The first tapered tract expands in cross sectional area from the entrance to the inlet port to the inlet ball check valve assembly, and the second tapered tract decreases in cross sectional area from the inlet ball check valve assembly to the chamber, and the inlet ball check valve assembly gates flow between the pumping chamber and the inlet port. Further, the third tapered tract expands in cross sectional area from the chamber to the outlet ball check valve assembly and the fourth tapered tract decreases in cross sectional area from the outlet ball check valve assembly to the exit from the outlet port and the outlet ball check valve assembly gates flow between the pumping chamber and the outlet port.

Alternatively, or in addition, a taper of at least one of the tapered tracts is conical. Also, alternatively or in addition, the pump-valving assembly further includes an inflow transition region, coupled between the second tapered tract and the chamber, configured to conduct fluid along a path that is roughly tangential to an internal circumference of the chamber, so as to smoothly establish fluid flow in the chamber.

Alternatively, or in addition, the pump-valving assembly further includes an outflow transition region, coupled between the chamber and the third tapered tract, providing a channel, beginning in the chamber, of gradually increasing width, the outflow transition region configured to allow fluid to smoothly exit from fluid rotation in the chamber to linear flow in the third tapered tract.

### Brief Description of the Drawings

The foregoing features of embodiments will be more readily understood by reference to the following detailed description, taken with reference to the accompanying drawings, in which:
Fig. **1** is a horizontal section of a pump-valving assembly **101** in accordance with an embodiment of the present invention, wherein the pump-valving assembly **101** is in diastole mode in which the chamber **102** is being filled.
Fig. **2** is a horizontal section of the pump-valving assembly **101** of Fig. **1****,** wherein the pump-valving assembly **101** is in systole mode in which the chamber **102** is being emptied.
Fig. **3** is a vertical section of the inlet ball check valve assembly **110** of the embodiment of Fig. **1** wherein the pump-valving assembly **101** is in diastole mode in which the chamber **102** is being filled and the inlet ball **114** is in the filling position.
Fig. **4** is a vertical section of the inlet ball check valve assembly **110** of the embodiment of Fig. **1** wherein the pump-valving assembly **101** is in a transition mode between the diastolic and systolic modes and the inlet ball **114** is in an unseated position.
Fig. **5** is a vertical section of the inlet ball check valve assembly **110** of the embodiment of Fig. **1** wherein the pump-valving assembly **101** is in systole mode in which the chamber **102** is being emptied and the inlet ball **114** is in the emptying position.
Fig. **6** is a vertical section of the outlet ball check valve assembly **120** of the embodiment of Fig. **1** wherein the pump-valving assembly **101** is in diastole mode in which the chamber **102** is being filled and the outlet ball **124** is in the filling position.
Fig. **7** is a vertical section of the outlet ball check valve assembly **120** of the embodiment of Fig. **1** wherein the pump-valving assembly **101** is in a transition mode between the diastolic and systolic modes and the outlet ball **124** is in an unseated position.
Fig. **8** is a vertical section of the outlet ball check valve assembly **120** of the embodiment of Fig. **1** wherein the pump-valving assembly **101** is in systole mode in which the chamber **102** is being emptied and the outlet ball **124** is in the emptying position.
Fig. **9** is an exploded perspective view, from above, of the pump-valving assembly **101** of Fig. **1****.**
Fig. **10A** is a side view of the inlet port **111** of the embodiment of Fig. **1****.**
Fig. **10B** is a vertical section of the inlet port **111** of Fig **10A** taken through the plane A-A.
Fig. **10C** is an end view of the inlet port **111** of Fig. **10A****.**
Fig. **10D** is a perspective view of the inlet port **111** of Fig. **10A****.**
Fig. **11A** is a side view of the outlet port **121** of the embodiment of Fig. **1****.**
Fig. **11B** is a vertical section of the outlet port **121** of Fig **11A** taken through the plane A-A.
Fig. **11C** is an end view of the outlet port **121** of Fig. **11A****.**
Fig. **11D** is a perspective view of the outlet port **121** of Fig. **11A****.**

### Detailed Description of Specific Embodiments

Definitions. As used in this description and the accompanying claims, the following terms shall have the meanings indicated, unless the context otherwise requires:
"Normal flow" is flow from the entrance to the inlet port **111** through the chamber **102** to the exit of the outlet port **121.**

A "slight reversal of flow" past a ball in a ball check valve is a small, controlled amount of desired reverse flow past the ball before the ball is seated in a closed position.

"Diastole mode" is a phase of operation of a pulsatile pump, according to embodiments of the present invention, during which the diaphragm (not shown) of the pump-valving assembly **101** is pulled away from the chamber **102** so as to create negative pressure within the chamber **102,** inlet ball check valve assembly **110,** and third tapered tract **126,** but not the fourth tapered tract **122.**

"Systole mode" is a phase of operation of a pulsatile pump, according to embodiments of the present invention, during which the diaphragm (not shown) is pushed towards the chamber **102,** so as to create positive pressure within the chamber **102,** outlet ball check valve assembly **120,** and the second tapered tract **116,** but not the first tapered tract **112.**

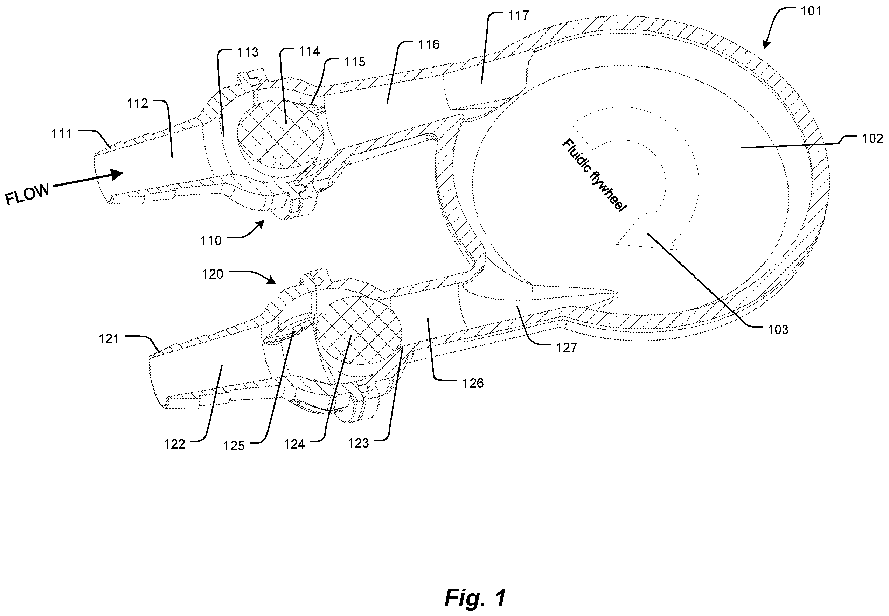

Fig. **1** is a horizontal section of a pump-valving assembly **101** in accordance with an embodiment of the present invention, wherein the pump-valving assembly **101** is in diastole mode in which the chamber **102** is being filled. Fluid flows into the inlet port **111,** through the first tapered tract **112,** past the inlet ball **114,** as that inlet ball **114** engages against the inlet ribs **115** that create gaps between the inlet ball **114** and the second tapered tract **116** that allow fluid to flow into the second tapered tract **116** and then into the chamber **102.** The pump-valving assembly **101** operates in cooperation with a diaphragm (not shown) that seats around the circumference of the chamber **102.** The motion of the diaphragm in cooperation with the inlet ball check valve assembly **110** and the outlet ball check valve assembly **120** causes the flow of fluid into the chamber **102.** While the chamber **102** is filling, the outlet ball **124** in the outlet ball check valve assembly **120** settles against the outlet seat **123** to prevent fluid flow from the outlet port **121** back into the chamber **102.** The fluidic flywheel **103** (described later) is illustrated. (In these figures, like numbered items correspond to similar components across different figures.)

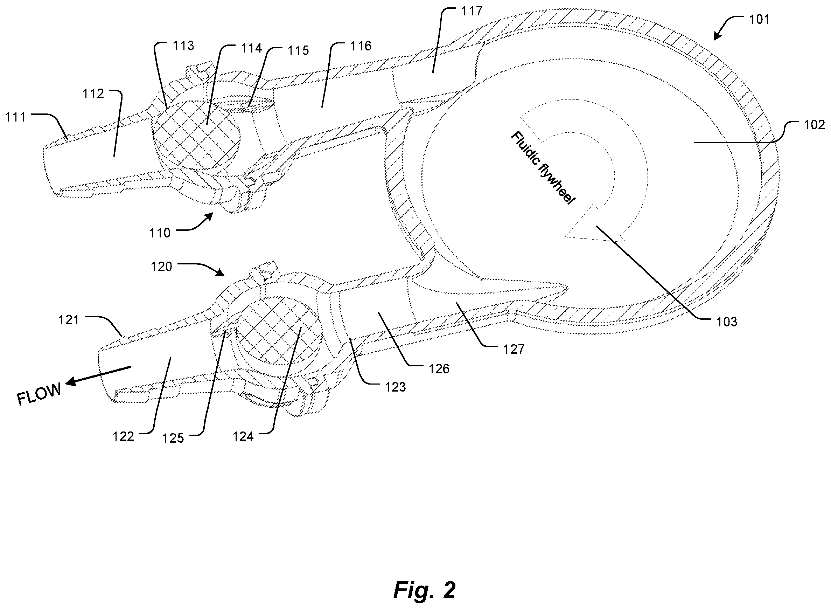

Fig. **2** is a horizontal section of the pump-valving assembly **101** of Fig. **1****,** wherein the pump-valving assembly **101** is in systole mode in which the chamber **102** is being emptied. The outlet port **121** receives fluid from the chamber **102** via the third tapered tract **126,** past the outlet ball **124** as that outlet ball **124** engages against the outlet ribs **125** that create gaps between the outlet ball **124** and the fourth tapered tract **122** that allow fluid to flow into the fourth tapered tract **122** and then exit the outlet port **121.** The pump-valving assembly **101** operates in cooperation with a diaphragm (not shown) that seats around the circumference of the chamber **102.** The motion of the diaphragm in cooperation with the inlet ball check valve assembly **110** and the outlet ball check valve assembly **120** causes flow of the fluid out of the chamber **102.** While the chamber **102** is emptying, the inlet ball **114** in the inlet ball check valve assembly **110** settles against inlet seat **113** to prevent fluid flow from the chamber **102** back into the inlet port **111.** The fluidic flywheel **103** (described later) is illustrated.

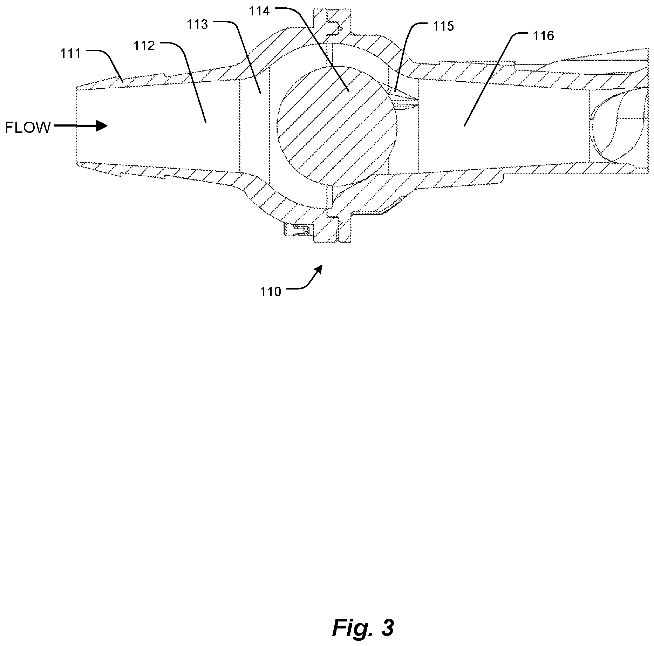

Fig. **3** is a vertical section of the inlet ball check valve assembly **110** of the embodiment of Fig. **1** wherein the pump-valving assembly **101** is in diastole mode in which the chamber **102** is being filled and the inlet ball **114** is in the filling position. The negative pressure created by the diaphragm (not shown) pulling away from the chamber **102** as it is filling causes fluid to flow into the inlet port **111,** through the first tapered tract **112,** past the inlet ball **114,** as that inlet ball **114** engages against the inlet ribs **115,** there result gaps between the inlet ball **114** and the second tapered tract **116,** so as to allow fluid to flow into the second tapered tract **116** and then into the chamber **102.**

Fluid flow in the tapered tracts **112, 116, 122,** and **126** provides a gradual change in velocity as a function of cross-sectional area to maintain continuity of flow leading up to and past each of the balls **114** and **124.** For either ball **114** or **124,** the ball **114** or **124** geometry, the physical properties of the ball **114** or **124,** and the ball check-valve assembly **110** or **120** as a whole are configured to stabilize the ball **114** or **124** in successive positions and transitions to these positions, as well as the velocity of the ball **114** or **124,** as these things are modulated in the course of pulsatile flow of fluid through the pump-over the ranges of viscosity for blood or other fluids being pumped. In the course of pumping, the pulsatile nature of the pump causes a change in direction of flow over the balls **114** and **124;** the pump is configured to achieve this change in direction in a manner that minimizes the total pressure loss across the ball check valve assembly **110** and **120.** Additionally, when a given one of the balls **114** or **124** has moved to an open position (against the ribs **115** or **125,** as the case may be) enabling flow past the given ball **114** or **124,** the pump is configured to maintain sufficient flow velocity (and differential pressure across the given ball **114** or **124)** to cause it to be retained against the ribs **115** or **125.** Similarly, the check valve assembly **110** and **120** is configured to create symmetry of other fluid velocity forces on the given ball **114** or **124** to prevent unwanted lateral motion of the given ball **114** or **124.**

The ribs **115** and **125** occupy a portion of the volume of the ball check valve assembly **110** or **120** in which they are located. That volume displaced by these ribs **115** or **125** therefore reduces the cross-sectional area of the ball check valve assembly **110** or **120** in the vicinity of the ribs **115** or **125** and thus causes an increase in velocity of fluid flow in the region. We utilize this increased velocity, as described in the previous paragraph, to cause the given ball **114** or **124** to be retained against the ribs **115** or **125.**

Although operation of the pump-valving assembly **101** is pulsatile in nature, the flow in the chamber **102** is always in a uniform direction (in this figure, clockwise). Fluid enters the chamber **102** ultimately from the inlet port **111** and leaves the chamber **102** ultimately through the outlet port **121.** This flow produces clockwise motion of the fluid in the chamber **102.** The clockwise motion is triggered at least in part by configuring the geometry of the pump-valving assembly **101** to introduce tangential flow of fluid from the inlet port **111** into the chamber **102.** Additionally, the large change in volume of the chamber **102,** caused by the diaphragm (not shown), in the course of pumping causes fluid to undergo multiple revolutions in the chamber **102** over each stroke of the diaphragm **202.** The fluid motion produces what we call a "fluidic flywheel" **103,** wherein the inertia of the fluid rotating in the chamber **102** engages with fluid entering from the inlet port **111** and thus continues the fluidic flywheel. Without the fluidic flywheel **103,** in-flowing fluid would encounter static resistance of fluid already in the chamber **102.** The fluidic flywheel **103** produces a relatively smooth flow of fluid through the pump from end to end.

The pump-valving assembly **101** includes a fluid inflow transition region **117** protruding into the chamber **102,** and a fluid outflow transition region **127** protruding from the chamber **102.** These transition regions **117** or **127** mediate between the circular flow in the chamber **102** and the linear flow in the tapered tracts **116** or **126,** respectively. The transition region **117** has the principal function of smoothly establishing circular flow in the chamber **102** and does so by conducting the fluid along a path that is roughly tangential to an internal circumference of the chamber **102.** In contrast, the transition area **127** has the somewhat more complex function of allowing fluid to smoothly exit from the fluidic flywheel in the chamber **102** and achieve a transition to linear flow in the third tapered tract **126** to enable flow through the outlet port **121,** and does so by providing a channel, beginning in the chamber **102,** of gradually increasing width.

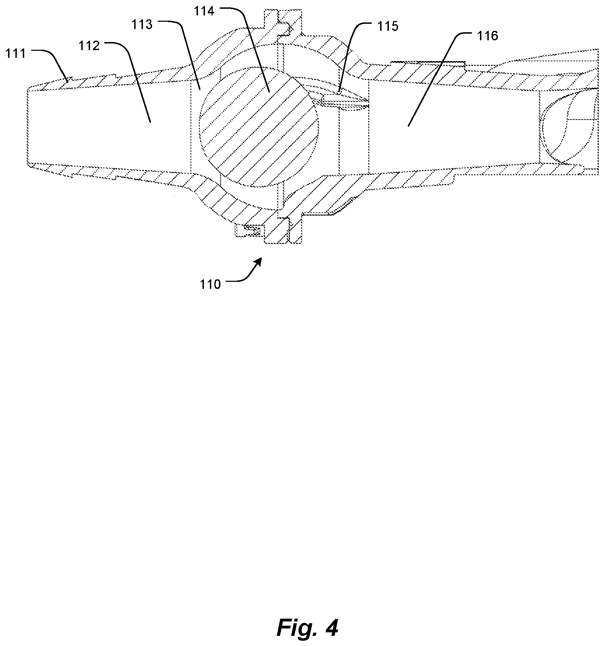

Fig. **4** is a vertical section of the inlet ball check valve assembly **110** of the embodiment of Fig. **1** wherein the pump-valving assembly **101** is in a transition mode between the diastolic and systolic modes and the inlet ball **114** is in an unseated position. During transition mode there is a slight reversal of flow until the inlet ball **114** settles against the inlet seat **113.**

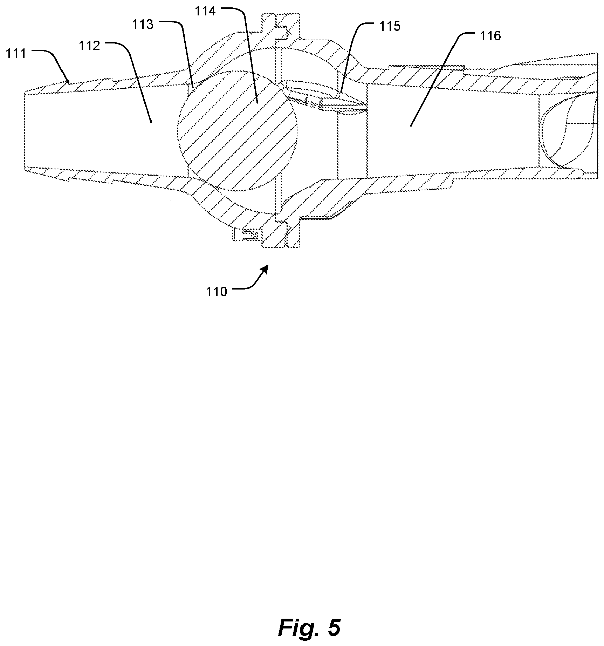

Fig. **5** is a vertical section of the inlet ball check valve assembly **110** of the embodiment of Fig. **1** wherein the pump-valving assembly **101** is in systole mode in which the chamber **102** is being emptied and the inlet ball **114** is in the emptying position. The positive pressure created by the diaphragm (not shown) pushing towards the chamber **102** as it is emptying causes the inlet ball **114** in the inlet ball check valve assembly **110** to settle against inlet seat **113,** so as to prevent fluid flow from the chamber **102** back into the inlet port **111.**

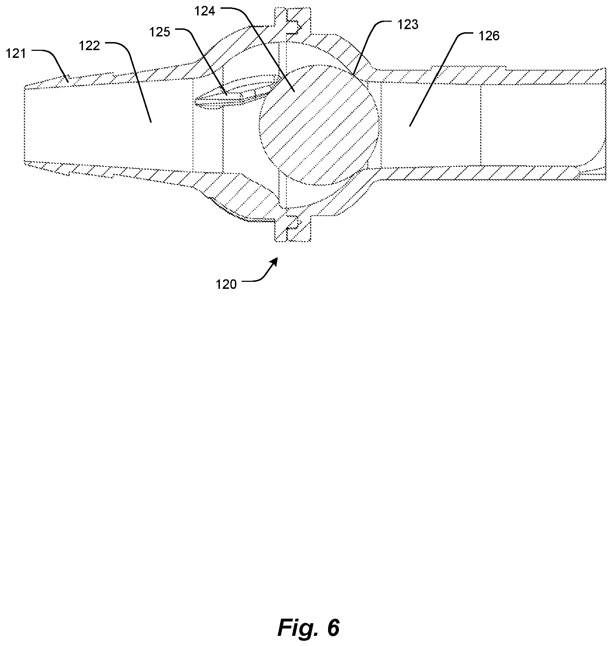

Fig. **6** is a vertical section of the outlet ball check valve assembly **120** of the embodiment of Fig. **1** wherein the pump-valving assembly **101** is in diastole mode in which the chamber **102** is being filled and the outlet ball **124** is in the filling position. The negative pressure created by the diaphragm (not shown) pulling away from the chamber **102** while it is filling causes the outlet ball **124** in the outlet ball check valve assembly **120** to settle against the outlet seat **123,** so as to prevent fluid flow from the outlet port **121** back into the chamber **102.**

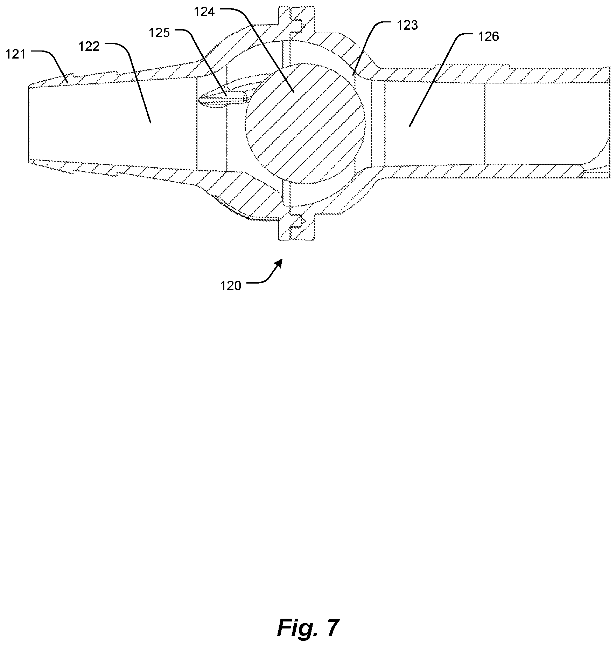

Fig. **7** is a vertical section of the outlet ball check valve assembly **120** of the embodiment of Fig. **1** wherein the pump-valving assembly **101** is in a transition mode between the diastolic and systolic modes and the outlet ball **124** is in an unseated position. During transition mode there is a slight reversal of flow until the outlet ball **124** settles against the outlet seat **123.**

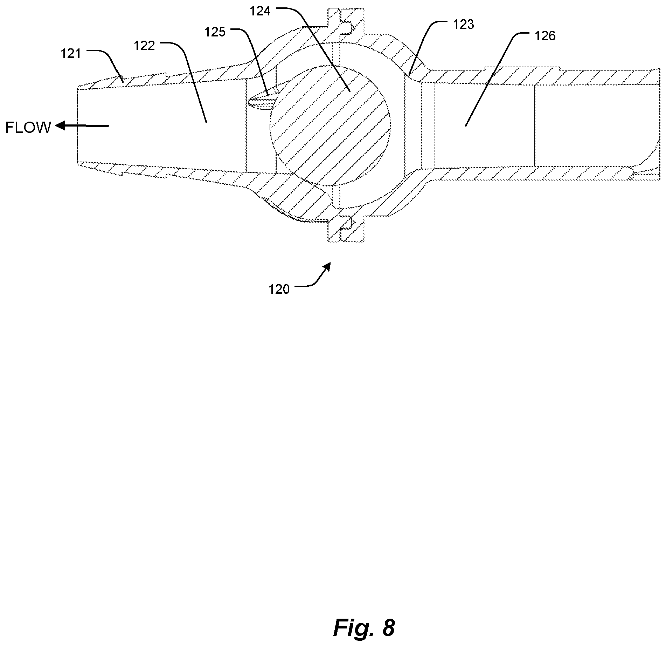

Fig. **8** is a vertical section of the outlet ball check valve assembly **120** of the embodiment of Fig. **1** wherein the pump-valving assembly **101** is in systole mode in which the chamber **102** is being emptied and the outlet ball **124** is in the emptying position. The positive pressure created by the diaphragm (not shown) pushing towards the chamber **102** as it is emptying causes the outlet ball **124** in the outlet ball check valve assembly **120** to settle against the outlet ribs **125,** so as to create gaps between the outlet ball **124** and the fourth tapered tract **122** and allow fluid to flow into the fourth tapered tract **122** and then exit the outlet port **121.**

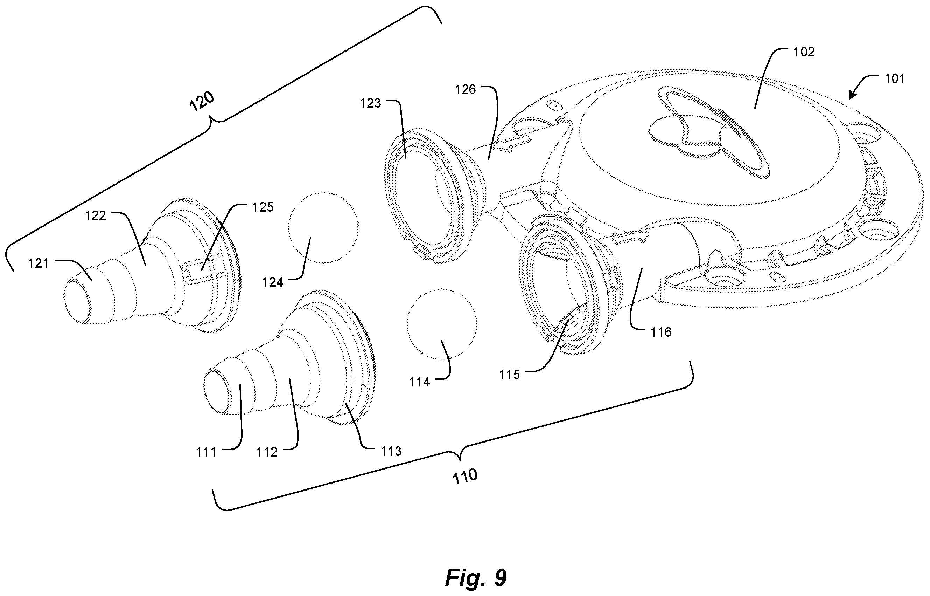

Fig. **9** is an exploded perspective view, from above, of the pump-valving assembly **101** of Fig. **1** showing the pump-valving assembly **101,** the chamber **102** (here covered), the inlet port assembly **110,** the inlet port **111,** first tapered tract **112,** inlet seat **113,** inlet ball **114,** the inlet ribs **115,** the second tapered tract **116,** outlet port assembly **120,** the outlet port **121,** the fourth tapered tract **122,** the outlet seat **123,** the outlet ball **124,** the outlet ribs **125,** and the third tapered tract **126.** The diaphragm (not shown) is attached around the circumference of the bottom of the chamber **102,** pushing up towards the chamber **102** in systole mode and pulling down away from the chamber **102** in diastole mode. In the pulsatile pumping of blood, although it might be thought that discontinuities in fluid flow may be useful, we have found generally that discontinuities in flow are undesirable. The pump of the present embodiment includes a number of features to reduce or eliminate such discontinuities. In particular, the tapered tracts **112, 116, 122,** and **126** are configured to reduce turbulence in inflow leading to, over, and beyond the inlet ball **114** and similarly in outflow leading to, over, and beyond the outlet ball **124.**

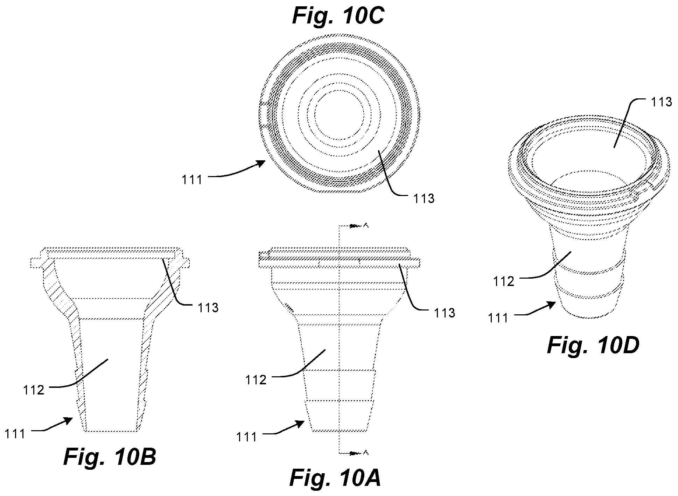

Fig. **10A** is a side view of the inlet port **111** of the embodiment of Fig. **1****.**

Fig. **10B** is a vertical section of the inlet port **111** of Fig **10A** taken through the plane A-A showing the first tapered tract **112,** and the inlet seat **113** against which the inlet ball 114 rests to occlude flow when the pump-valving assembly **101** is in systole mode.

Fig. **10C** is an end view of the inlet port **111** of Fig. **10A** showing the first tapered tract **112** and the inlet seat **113.**

Fig. **10D** is a perspective view of the inlet port **111** of Fig. **10A****.**

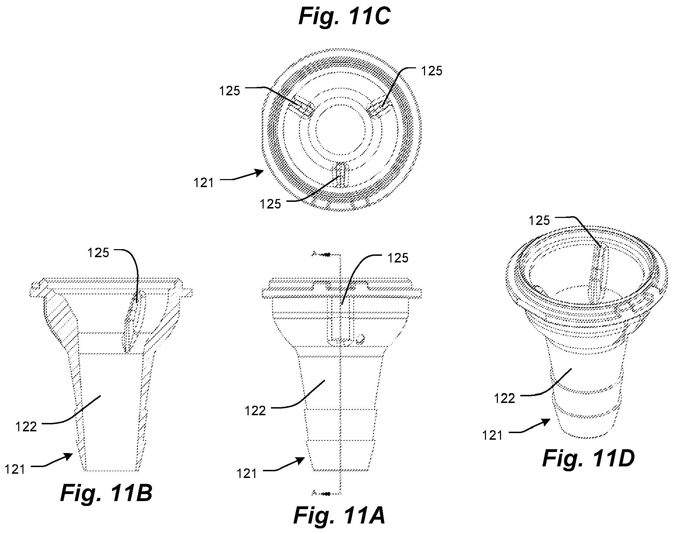

Fig. **11A** is a side view of the outlet port **121** of the embodiment of Fig. **1****.**

Fig. **11B** is a vertical section of the outlet port **121** of Fig **11A** taken through the plane A-A showing the fourth tapered tract **122,** and the outlet ribs **125** that create gaps that allow normal fluid flow when the pump-valving assembly **101** is in systole mode.

Fig. **11C** is an end view of the outlet port **121** of Fig. **11A** showing the fourth tapered tract **122,** and the outlet ribs **125.**

Fig. **11D** is a perspective view of the outlet port **121** of Fig. **11A****.**

The structure of a pulsatile pump in accordance with various embodiments of the present invention can usefully reflect attributes of the human heart. The human heart is preload sensitive-the heart cannot "pull" blood into the left ventricle; it can only allow the blood available to flow naturally into the ventricle. The human heart is also afterload sensitive in that it is responsive to the compliance and resistance in the downstream vasculature and doesn't exert excess force on the blood, which could damage the vasculature. Lastly, the left ventricle cannot deliver blood that isn't in the ventricle when it contracts; there is a limited bolus of blood that it can deliver.

The pump-valving assembly **101** has similar attributes of inherent safety: it is preload and afterload sensitive, and it is limited in both the volume of blood it can deliver and the force at which it can deliver that bolus of blood. When filling, the pump-valving assembly **101** allows gravity filling from the venous reservoir, exerting minimal negative pressure. When the chamber in the pump-valving assembly **101** is emptying, the linear motor that powers the pump valving assembly is limited by design. In consequence the pump valving assembly cannot overpressure the downstream tubing or vasculature, instead delivering less than the volume of blood in the pump chamber **102,** thereby only delivering as much volume as the vasculature can receive.

The pump-valving assembly **101** is analogous to a left ventricle of the human heart; the inlet ball check valve assembly **110** used in various embodiments hereof is analogous to a mitral valve; and the outlet ball check valve assembly**120** used in various embodiments hereto is analogous to an aortic valve. Like the human heart, the inlet **110** and outlet **120** ball check valve assemblies are passive and require a slight reversal of flow to close. This slight reversal of flow mimics the slight reversal that occurs when the aortic valve of the human heart closes.

The embodiments of the invention described above are intended to be merely exemplary; numerous variations and modifications will be apparent to those skilled in the art. Variations and modifications are intended to be within the scope of the present invention as defined in any appended claims.

## Claims

1. . A pump-valving assembly (101) for a pulsatile fluid pump, the assembly comprising:
a generally cylindrical pumping chamber (102) defined by an exterior wall;
an inlet port (111) having an entrance and coupled to the pumping chamber (102); and
an outlet port (121) having an exit and coupled to the pumping chamber (102);
a spherically shaped inlet ball check-valve assembly (110) coupled to the inlet port (111) through a first tapered tract (112), distinct from the inlet ball check-valve assembly (110), and to the pumping chamber (102) through a second tapered tract (116), distinct from the inlet ball check-valve assembly (110), the second tapered tract (116) extending directly to the exterior wall of the pumping chamber (102), the inlet ball check-valve assembly (110) having an interior wall defining a volume in which an inlet ball (114) is disposed, the assembly including an inlet seat and inlet ribs (115) configured to engage the inlet ball (114);
a spherically shaped outlet ball check-valve assembly (120) coupled to the pumping chamber (102) through a third tapered tract, distinct from the outlet ball check-valve assembly, the third tapered tract extending directly to the exterior wall of the pumping chamber and to the outlet port through a fourth tapered tract, distinct from the outlet ball check-valve assembly, the outlet ball check-valve assembly having an interior wall defining a volume in which an outlet ball (124) is disposed, the assembly including an outlet seat and outlet ribs (125) configured to engage the outlet ball (124);
so that:
(i) the first tapered tract expands in cross sectional area from the entrance to the inlet port to the inlet ball check valve assembly, and the second tapered tract decreases in cross sectional area from the inlet ball check valve assembly to the chamber, and the inlet ball check valve assembly gates flow between the pumping chamber and the inlet port, and
(ii) the third tapered tract expands in cross sectional area from the chamber to the outlet ball check valve assembly and the fourth tapered tract decreases in cross sectional area from the outlet ball check valve assembly to the exit from the outlet port and the outlet ball check valve assembly gates flow between the pumping chamber and the outlet port.

2. . A pump-valving assembly according to claim 1, wherein a taper of at least one of the tapered tracts is conical.

3. . A pump-valving assembly according to claim 1, further comprising an inflow transition region, coupled between the second tapered tract and the chamber, configured to conduct fluid along a path that is roughly tangential to an internal circumference of the chamber, so as to smoothly establish circular fluid flow in the chamber.

4. . A pump-valving assembly according to claim 1, further comprising an outflow transition region, coupled between the chamber and the third tapered tract, providing a channel, beginning in the chamber, of gradually increasing width, the outflow transition region configured to allow fluid to smoothly exit from fluid rotation in the chamber to linear flow in the third tapered tract.

5. . A pump-valving assembly according to claim 3, further comprising an outflow transition region, coupled between the chamber and the third tapered tract, providing a channel, beginning in the chamber, of gradually increasing width, the outflow transition region configured to allow fluid to smoothly exit from fluid rotation in the chamber to linear flow in the third tapered tract.

## Patentansprüche

1. Pumpenventilbaugruppe (101) für eine pulsierende Fluidpumpe, die Baugruppe umfassend:
eine im Allgemeinen zylindrische Pumpenkammer (102), die durch eine Außenwand definiert ist;
einen Einlassanschluss (111), der einen Eingang aufweist und mit der Pumpenkammer (102) gekoppelt ist; und
einen Auslassanschluss (121), der einen Ausgang aufweist und mit der Pumpenkammer (102) gekoppelt ist;
eine kugelförmige Einlasskugelrückschlagventilbaugruppe (110), die mit dem Einlassanschluss (111) über einen ersten, von der Einlasskugelrückschlagventilbaugruppe (110) unterschiedlichen, verjüngten Kanal (112) und mit der Pumpkammer (102) über einen zweiten, von der Einlasskugelrückschlagventilbaugruppe (110) unterschiedlichen, verjüngten Kanal (116) gekoppelt ist, der zweite verjüngte Kanal (116) sich direkt zur Außenwand der Pumpkammer (102) erstreckt, wobei die Einlasskugelrückschlagventilbaugruppe (110) eine Innenwand aufweist, die ein Volumen definiert, in dem eine Einlasskugel (114) angeordnet ist, wobei die Baugruppe einen Einlasssitz und Einlassrippen (115) aufweist, die konfiguriert sind, um mit der Einlasskugel (114) in Eingriff zu kommen;
eine kugelförmige Auslasskugelrückschlagventilbaugruppe (120), die mit der Pumpkammer (102) über einen dritten verjüngten Abschnitt gekoppelt ist, der sich von der Auslasskugelrückschlagventilbaugruppe unterscheidet, wobei sich der dritte verjüngte Abschnitt direkt zu der Außenwand der Pumpkammer und zu dem Auslassanschluss über einen vierten verjüngten Abschnitt erstreckt, anders als die Auslasskugelrückschlagventilbaugruppe, wobei die Auslasskugelrückschlagventilbaugruppe eine Innenwand aufweist, die ein Volumen definiert, in dem eine Auslasskugel (124) angeordnet ist, wobei die Baugruppe einen Auslasssitz und Auslassrippen (125) aufweist, die konfiguriert sind, um die Auslasskugel (124) in Eingriff zu nehmen;
sodass:
(i) der erste verjüngte Abschnitt sich vom Eingang des Einlassanschlusses bis zur Einlasskugelrückschlagventilbaugruppe im Querschnittsbereich vergrößert und der zweite verjüngte Abschnitt sich von der Einlasskugelrückschlagventilbaugruppe bis zur Kammer im Querschnittsbereich verkleinert und die Einlasskugelrückschlagventilbaugruppe den Durchfluss zwischen der Pumpkammer und dem Einlassanschluss sperrt,
und
(ii) der dritte verjüngte Abschnitt sich im Querschnittsbereich von der Kammer bis zur Auslasskugelrückschlagventilbaugruppe vergrößert und der vierte verjüngte Abschnitt sich im Querschnittsbereich von der Auslasskugelrückschlagventilbaugruppe bis zum Austritt aus der Auslassöffnung verkleinert und die Auslasskugelrückschlagventilbaugruppe den Durchfluss zwischen der Pumpkammer und dem Auslassanschluss freigibt.

2. Pumpenventilbaugruppe nach Anspruch 1, wobei mindestens einer der sich verjüngenden Trakte konisch ist.

3. Pumpenventilbaugruppe nach Anspruch 1, ferner umfassend eine Einströmungsübergangsregion, die zwischen den zweiten verjüngten Trakt und die Kammer gekoppelt ist und konfiguriert ist, um Fluid entlang eines Pfades zu leiten, der in etwa tangential zu einem Innenumfang der Kammer verläuft, um einen kreisförmigen Fluidfluss in der Kammer gleichmäßig herzustellen.

4. Pumpenventilbaugruppe nach Anspruch 1, ferner umfassend eine Ausflussübergangsregion, die zwischen die Kammer und den dritten verjüngten Trakt gekoppelt ist und einen in der Kammer beginnenden Kanal mit allmählich zunehmender Breite bereitstellt, wobei die Ausflussübergangsregion konfiguriert ist, um dem Fluid einen reibungslosen Übergang von der Fluiddrehung in der Kammer zu einer linearen Strömung in dem dritten verjüngten Trakt zu ermöglichen.

5. Pumpenventilbaugruppe nach Anspruch 3, ferner umfassend eine Ausflussübergangsregion, die zwischen die Kammer und den dritten verjüngten Trakt gekoppelt ist und einen in der Kammer beginnenden Kanal mit allmählich zunehmender Breite bereitstellt, wobei die Ausflussübergangsregion konfiguriert ist, um dem Fluid einen reibungslosen Übergang von der Fluiddrehung in der Kammer zu einer linearen Strömung in dem dritten verjüngten Trakt zu ermöglichen.

## Revendications

1. . Ensemble pompe-soupape (101) pour pompe à fluide pulsatile, l'ensemble comprenant :
une chambre de pompage généralement cylindrique (102) définie par une paroi extérieure ;
un orifice d'entrée (111) ayant une entrée et étant couplé à la chambre de pompage (102) ; et
un orifice de sortie (121) ayant une sortie et étant couplé à la chambre de pompage (102) ;
un ensemble clapet de non-retour à bille d'entrée de forme sphérique (110) couplé à l'orifice d'entrée (111) via une première voie effilée (112), distincte de l'ensemble clapet de non-retour à bille d'entrée (110), et à la chambre de pompage (102) via une deuxième voie effilée (116), distincte de l'ensemble clapet de non-retour à bille d'entrée (110), la deuxième voie effilée (116) se prolongeant directement jusqu'à la paroi extérieure de la chambre de pompage (102), l'ensemble clapet de non-retour à bille d'entrée (110) ayant une paroi intérieure définissant un volume dans lequel une bille d'entrée (114) est disposée, l'ensemble comportant un siège d'entrée et des nervures d'entrée (115) configurées pour mettre en prise la bille d'entrée (114) ;
un ensemble clapet de non-retour à bille de sortie de forme sphérique (120) couplé à la chambre de pompage (102) via une troisième voie effilée, distincte de l'ensemble clapet de non-retour à bille de sortie, la troisième voie effilée se prolongeant directement vers la paroi extérieure de la chambre de pompage et vers l'orifice de sortie via une quatrième voie effilée, distincte de l'ensemble clapet de non-retour à bille de sortie, l'ensemble clapet de non-retour à bille de sortie ayant une paroi intérieure définissant un volume dans lequel une bille de sortie (124) est disposée, l'ensemble comportant un siège de sortie et des nervures de sortie (125) configurées pour mettre en prise la bille de sortie (124) ;
de sorte que :
(i) la première voie effilée se dilate en section transversale de l'entrée à l'orifice d'entrée jusqu'à l'ensemble clapet de non-retour à bille d'entrée, et la deuxième voie effilée diminue en section transversale de l'ensemble clapet de non-retour à bille d'entrée jusqu'à la chambre, et l'ensemble clapet de non-retour à bille d'entrée assure l'écoulement entre la chambre de pompage et l'orifice d'entrée, et
(ii) la troisième voie effilée se dilate en section transversale de la chambre à l'ensemble clapet de non-retour à bille de sortie et la quatrième voie effilée diminue en section transversale de l'ensemble clapet de non-retour à bille de sortie à la sortie de l'orifice de sortie et l'ensemble clapet de non-retour à bille de sortie assure l'écoulement entre la chambre de pompage et l'orifice de sortie.

2. . Ensemble pompe-soupape selon la revendication 1, dans lequel une partie effilée d'au moins l'une des voies effilées est conique.

3. . Ensemble pompe-soupape selon la revendication 1, comprenant en outre une région de transition d'entrée, couplée entre la deuxième voie effilée et la chambre, configurée pour conduire le fluide le long d'un trajet qui est approximativement tangentiel à une circonférence interne de la chambre, de manière à établir en douceur un écoulement de fluide circulaire dans la chambre.

4. . Ensemble pompe-soupape selon la revendication 1, comprenant en outre une région de transition de sortie, couplée entre la chambre et la troisième voie effilée, fournissant un canal, commençant dans la chambre, de largeur augmentant progressivement, la région de transition de sortie étant configurée pour permettre au fluide de sortir en douceur de la rotation du fluide dans la chambre vers un écoulement linéaire dans la troisième voie effilée.

5. . Ensemble pompe-soupape selon la revendication 3, comprenant en outre une région de transition de sortie, couplée entre la chambre et la troisième voie effilée, fournissant un canal, commençant dans la chambre, de largeur augmentant progressivement, la région de transition de sortie étant configurée pour permettre au fluide de sortir en douceur de la rotation du fluide dans la chambre vers un écoulement linéaire dans la troisième voie effilée.
